# EUROPEAN PATENT SPECIFICATION

(11) **EP 3 477 758 B1**
(45) Date of publication and mention of the grant of the patent: **16.12.2020**
(21) Application number: 17830940.7
(22) Date of filing: 13.07.2017
(51) Int. Cl.: H01M 10/0567, H01G 11/64, H01M 10/0525

(54) **NONAQUEOUS ELECTROLYTE, POWER STORAGE ELEMENT, AND METHOD FOR PRODUCING POWER STORAGE ELEMENT**
WASSERFREIER ELEKTROLYT, ENERGIESPEICHERELEMENT UND VERFAHREN ZUR HERSTELLUNG EINES ENERGIESPEICHERELEMENTS
ÉLECTROLYTE NON AQUEUX, ÉLÉMENT DE STOCKAGE D'ÉNERGIE ET PROCÉDÉ DE FABRICATION D'ÉLÉMENT DE STOCKAGE D'ÉNERGIE

(30) Priority: 22.07.2016 JP 2016144441
(43) Date of publication of application: 01.05.2019
(73) Proprietor: GS Yuasa International Ltd., Kyoto-shi, Kyoto 601-8520 (JP)
(72) Inventor: HACHIDA, Takeshi, Kyoto-shi Kyoto 601-8520 (JP); NISHIE, Katsushi, Kyoto-shi Kyoto 601-8520 (JP)
(74) Representative: Isarpatent
(86) International application number: PCT/JP2017/025619
(87) International publication number: WO 2018/016421

(56) References cited:
- WO-A1-2015/133097
- JP-A- S58 214 281
- JP-A- 2001 332 296
- JP-A- 2007 518 229
- JP-A- 2009 164 130
- US-A- 3 982 958

## Description

### TECHNICAL FIELD

The present invention relates to a nonaqueous electrolyte, an energy storage device, and a method for producing an energy storage device.

### BACKGROUND ART

Nonaqueous electrolyte secondary batteries typified by lithium ion secondary batteries are widely used in electronic apparatuses such as personal computers and communication terminals, automobiles and the like because of their high energy density. In general, the nonaqueous electrolyte secondary battery includes an electrode assembly having a pair of electrodes electrically isolated by a separator and a nonaqueous electrolyte interposed between the electrodes, and is configured so as to charge and discharge by exchange of ions between both electrodes. Also, capacitors such as lithium ion capacitors and electric double layer capacitors are widely used as an energy storage device other than secondary batteries.

Various additives are added to the nonaqueous electrolyte used for such energy storage devices for the purpose of improving performance and the like. Specifically, a nonaqueous electrolyte to which fluorotoluene or the like is added for preventing overcharge has been proposed (see Patent Document 1).

### PRIOR ART DOCUMENT

### PATENT DOCUMENT

Patent Document 1: JP-A-2006-108100
Further, WO 2015/133097 A1 discloses a non-aqueous electrolyte secondary battery using a nonaqueous electrolyte containing a monofluorotoluene.

### SUMMARY OF THE INVENTION

### PROBLEMS TO BE SOLVED BY THE INVENTION

However, in the case of using a nonaqueous electrolyte to which halogenated toluene such as fluorotoluene is added, an inconvenience that swelling of the energy storage device (so-called battery swelling) is likely to be caused by repeated charge-discharge.

The present invention has been made based on the above-mentioned circumstances, and an object of the present invention is to provide a nonaqueous electrolyte capable of suppressing swelling of an energy storage device caused by repeated charge-discharge, an energy storage device including the nonaqueous electrolyte, and a method for producing the energy storage device.

### MEANS FOR SOLVING THE PROBLEMS

One aspect of the present invention that has been made to solve the above-mentioned problem is a nonaqueous electrolyte for an energy storage device in which an electrolyte salt is dissolved in a nonaqueous solvent and which further contains halogenated toluene and halogenated nitrotoluene, wherein the content of the halogenated toluene is 0.1% by mass or more and the content of the halogenated nitrotoluene is 0.001% by mass or more based on a total mass of the nonaqueous electrolyte.

Another aspect of the present invention is an energy storage device including the nonaqueous electrolyte.

Another aspect of the present invention is a method for producing an energy storage device, the method including a step of housing a positive electrode and a negative electrode in a case, and a step of injecting the nonaqueous electrolyte into the case.

### ADVANTAGES OF THE INVENTION

According to the present invention, it is possible to provide a nonaqueous electrolyte capable of suppressing swelling of an energy storage device caused by repeated charge-discharge, an energy storage device including the nonaqueous electrolyte, and a method for producing the energy storage device.

### BRIEF DESCRIPTION OF THE DRAWINGS

Fig. 1 is an external perspective view showing a secondary battery according to an embodiment of the present invention.
Fig. 2 is a schematic diagram showing an energy storage apparatus constituted by assembling a plurality of secondary batteries according to the embodiment of the present invention.

### MODE FOR CARRYING OUT THE INVENTION

One aspect of the present invention is a nonaqueous electrolyte which is used for an energy storage device and contains halogenated toluene and halogenated nitrotoluene. According to the nonaqueous electrolyte, it is possible to suppress swelling of the energy storage device caused by repeated charge-discharge. Although the reason why such effect occurs is not clear, it is assumed that the coexistence of the halogenated nitrotoluene and the halogenated toluene forms a coating on a positive electrode and decomposition of the nonaqueous electrolyte is suppressed, and consequently, the swelling of the energy storage device is suppressed. In addition, according to the nonaqueous electrolyte, it is also possible to increase the discharge capacity retention ratio of the energy storage device. Although the reason for this is also not clear, it is assumed that the action of suppressing decomposition of the nonaqueous electrolyte by the formed coating acts in the direction of increasing the discharge capacity retention ratio.

The halogenated toluene is preferably fluorotoluene. By using fluorotoluene, it is possible to more effectively suppress the swelling of the energy storage device, and the like.

The content of the halogenated toluene is 0.1% by mass or more and preferably 8% by mass or less based on the total mass of the nonaqueous electrolyte. By setting the content of the halogenated toluene in the above range, it is possible to increase the discharge capacity retention ratio while sufficiently suppressing the swelling of the energy storage device.

It is preferable that the halogenated nitrotoluene is fluoronitrotoluene. By using fluoronitrotoluene, it is possible to more effectively suppress the swelling of the energy storage device, and the like.

The content of the halogenated nitrotoluene is 0.001% by mass or more and preferably 3% by mass or less based on the total mass of the nonaqueous electrolyte. By setting the content of the halogenated nitrotoluene in the above range, it is possible to more effectively suppress the swelling of the energy storage device, and the like.

Another aspect of the present invention is an energy storage device including the nonaqueous electrolyte. Since the energy storage device includes the nonaqueous electrolyte, swelling is suppressed. In addition, the energy storage device can exhibit a good discharge capacity retention ratio.

Another aspect of the present invention is a method for producing an energy storage device, which uses the nonaqueous electrolyte. According to the method for producing an energy storage device, since the nonaqueous electrolyte is used, it is possible to suppress swelling and obtain an energy storage device having high discharge capacity retention ratio.

### <Nonaqueous Electrolyte>

A nonaqueous electrolyte according to one embodiment of the present invention is used for an energy storage device and contains halogenated toluene and halogenated nitrotoluene. The nonaqueous electrolyte is one in which an electrolyte salt is dissolved in a nonaqueous solvent and further contains halogenated toluene in an amount of 0.1% by mass or more and halogenated nitrotoluene in an amount of 0.001% by mass or more based on a total mass of the nonaqueous electrolyte.

### (Halogenated Toluene)

The halogenated toluene refers to a compound in which a part or all of hydrogen atoms of toluene are substituted with halogen atoms. Halogenated toluene may be used alone or two or more kinds may be mixed and used.

Examples of the halogen atom include a fluorine atom, a chlorine atom, a bromine atom and the like, and a fluorine atom is preferable. When the halogen atom is a fluorine atom, it is possible to further suppress the swelling of the energy storage device and also increase the discharge capacity retention ratio.

The number of halogen atoms in the halogenated toluene is not particularly limited, and is, for example, 1 or more and 4 or less, preferably 1 and 2, and more preferably 1.
When one halogenated toluene has a plurality of halogen atoms, plural kinds of halogen atoms may be the same or different.

The halogenated toluene is preferably halogenated toluene in which a hydrogen atom on a benzene ring (aromatic ring) is substituted with a halogen atom. In the case of such halogenated toluene, the bonding position of the halogen atom is preferably ortho and meta, and more preferably ortho to a methyl group.

Specific examples of the halogenated toluene include fluorotoluene (o-fluorotoluene, m-fluorotoluene, p-fluorotoluene, α-fluorotoluene), chlorotoluene, bromotoluene, difluorotoluene, dichlorotoluene, dibromotoluene, trifluorotoluene, trichlorotoluene, tribromotoluene, chlorofluorotoluene, bromofluorotoluene, and the like.

Among them, fluorotoluene is preferable, o-fluorotoluene (2-fluorotoluene) and m-fluorotoluene (3-fluorotoluene) are preferable, and o-fluorotoluene is more preferable. By using such halogenated toluene, it is possible to further suppress the swelling of the energy storage device and increase the discharge capacity retention ratio.

The mass (content) of the halogenated toluene based on the total mass of the nonaqueous electrolyte is 0.1% by mass, more preferably 0.5% by mass, further preferably 1% by mass, still more preferably 2% by mass, and particularly preferably 3% by mass. By setting the content of the halogenated toluene to the lower limit or more, it is possible to sufficiently exhibit the effect of using halogenated toluene as an overcharge preventing additive and the effect of maintaining the discharge capacity retention ratio. On the other hand, the upper limit of the mass (content) of the halogenated toluene based on the total mass of the nonaqueous electrolyte is preferably 8% by mass, and more preferably 6% by mass, from the viewpoint of various properties such as conductivity.

### (Halogenated Nitrotoluene)

The halogenated nitrotoluene refers to a compound in which a part or all of hydrogen atoms of nitrotoluene are substituted with halogen atoms. Halogenated nitrotoluene may be used alone or two or more kinds may be mixed and used.

Specific examples of the halogen atom are as described above. The halogen atom of the halogenated nitrotoluene is preferably a fluorine atom. When the halogen atom of the halogenated nitrotoluene is a fluorine atom, it is possible to further suppress the swelling of the energy storage device, increase the discharge capacity retention ratio, and the like.

The number of halogen atoms in the halogenated nitrotoluene is not particularly limited, and is, for example, 1 or more and 4 or less, preferably 1 and 2, and more preferably 1. When one halogenated nitrotoluene has a plurality of halogen atoms, plural kinds of halogen atoms may be the same or different.

The halogenated nitrotoluene is preferably halogenated nitrotoluene in which a hydrogen atom on a benzene ring (aromatic ring) is substituted with a halogen atom. In the case of such halogenated nitrotoluene, the bonding position of the halogen atom is preferably ortho and para, and more preferably ortho to a methyl group.

The number of nitro groups in the halogenated nitrotoluene is not particularly limited, and is usually 1. Also, the bonding position of the nitro group is preferably ortho and para, and more preferably ortho to a methyl group.

Specific examples of the halogenated toluene include fluoronitrotoluene, chloronitrotoluene, bromonitrotoluene, difluoronitrotoluene, dichloronitrotoluene, dibromonitrotoluene, trifluoronitrotoluene, trichloronitrotoluene, tribromonitrotoluene, chlorofluoronitrotoluene, bromofluoronitrotoluene, and the like.

Among them, fluoronitrotoluene is preferable. By using fluoronitrotoluene, it is possible to more effectively suppress the swelling of the energy storage device, and the like.

Examples of the fluoronitrotoluene include 2-fluoro-3-nitrotoluene, 2-fluoro-4-nitrotoluene, 2-fluoro-5-nitrotoluene, 2-fluoro-6-nitrotoluene, 3-fluoro-2-nitrotoluene, 3-fluoro-4-nitrotoluene, 4-fluoro-2-nitrotoluene, 4-fluoro-3-nitrotoluene, and the like. Among them, 2-fluoro-4-nitrotoluene and 2-fluoro-6-nitrotoluene are more preferable, and 2-fluoro-6-nitrotoluene is further preferable. By using such fluoronitrotoluene, it is possible to more effectively suppress the swelling of the energy storage device and also increase the retention ratio of the discharge capacity.

The mass (content) of the halogenated nitrotoluene based on the total mass of the nonaqueous electrolyte is 0.001% by mass, more preferably 0.005% by mass, further preferably 0.01% by mass, still more preferably 0.05% by mass, and particularly preferably 0.08% by mass. By setting the content of the halogenated nitrotoluene to the lower limit or more, it is possible to further suppress the swelling of the energy storage device and also increase the discharge capacity retention ratio. On the other hand, the upper limit of the mass (content) of the halogenated nitrotoluene based on the total mass of the nonaqueous electrolyte is preferably 3% by mass, more preferably 1% by mass, further preferably 0.5% by mass, and still more preferably 0.2% by mass. By setting the content of halogenated nitrotoluene to the upper limit or less, it is possible to further suppress the swelling of the energy storage device.

The lower limit of the content of the halogenated nitrotoluene based on 100 parts by mass of the halogenated toluene in the nonaqueous electrolyte is preferably 0.01 parts by mass, more preferably 0.1 parts by mass, further more preferably 0.5 parts by mass, still more preferably 1 part by mass, and still more preferably 1.5 parts by mass. On the other hand, the upper limit is preferably 10 parts by mass, more preferably 5 parts by mass, and further preferably 3 parts by mass. By setting the mass ratio of the halogenated toluene to the halogenated nitrotoluene in the above range, it is possible to more effectively suppress the swelling of the energy storage device by effectively forming a coating.

### (Nonaqueous Solvent)

As the nonaqueous solvent, a known nonaqueous solvent usually used as a nonaqueous solvent in a nonaqueous electrolyte of a general energy storage device can be used. Examples of the nonaqueous solvent include cyclic carbonate, linear carbonate, ester, ether, amide, sulfone, lactone, nitrile, and the like. Among them, it is preferable to use at least a cyclic carbonate or a chain carbonate, and it is more preferable to use a cyclic carbonate and a chain carbonate in combination. When a cyclic carbonate and a chain carbonate are used in combination, the volume ratio of the cyclic carbonate to the chain carbonate (cyclic carbonate : chain carbonate) is not particularly limited, and it is, for example, preferably 5 : 95 or more and 50 : 50 or less.

Examples of the cyclic carbonate include ethylene carbonate (EC), propylene carbonate (PC), butylene carbonate (BC), vinylene carbonate (VC), vinylethylene carbonate (VEC), chloroethylene carbonate, fluoroethylene carbonate (FEC), difluoroethylene carbonate (DFEC), styrene carbonate, catechol carbonate, 1-phenylvinylene carbonate, 1,2-diphenylvinylene carbonate, and the like, among which EC is preferable.

Examples of the chain carbonate include diethyl carbonate (DEC), dimethyl carbonate (DMC), ethyl methyl carbonate (EMC), diphenyl carbonate, and the like, among which EMC is preferable.

### (Electrolyte Salt)

As the electrolyte salt, a known electrolyte salt usually used as an electrolyte salt in a nonaqueous electrolyte of a general energy storage device can be used. Examples of the electrolyte salt include lithium salts, sodium salts, potassium salts, magnesium salts, onium salts and the like, and lithium salts are preferable.

Examples of the lithium salt include inorganic lithium salts such as LiPF6, LiPO2F2, LiBF4, LiClO4 and LiN(SO2F)2, lithium salts having a fluorinated hydrocarbon group such as LiSO3CF3, LiN(SO2CF3)2, LiN(SO2C2F5)2, LiN(SO2CF3)(SO2C4F9), LiC(SO2CF3)3 and LiC(SO2C2F5)3, and the like. Among them, an inorganic lithium salt is preferable, and LiPF6 is more preferable.

The lower limit of the content of the electrolyte salt in the nonaqueous electrolyte is preferably 0.1 M, more preferably 0.3 M, further preferably 0.5 M, and particularly preferably 0.7 M. On the other hand, the upper limit is not particularly limited, and is preferably 2.5 M, more preferably 2 M, and further preferably 1.5 M.

The nonaqueous electrolyte may contain other components other than the halogenated toluene, the halogenated nitrotoluene, the nonaqueous solvent and the electrolyte salt as long as the effect of the present invention is not impaired. Examples of the other components may include various additives contained in a nonaqueous electrolyte of a general energy storage device. However, the content of these other components may be preferably 5% by mass or less, and more preferably 1% by mass or less.

The nonaqueous electrolyte can be obtained by adding the electrolyte salt, halogenated toluene and halogenated nitrotoluene to the nonaqueous solvent and dissolving them.

### <Energy Storage Device>

An energy storage device according to an embodiment of the present invention includes a positive electrode, a negative electrode, and a nonaqueous electrolyte. Hereinafter, a nonaqueous electrolyte secondary battery will be described as an example of the energy storage device. The positive electrode and the negative electrode usually form an electrode assembly which is alternately superimposed by lamination or winding via a separator. The electrode assembly is housed in a case, and the nonaqueous electrolyte is filled in the case. In the secondary battery (energy storage device), the nonaqueous electrolyte described above is used as the nonaqueous electrolyte. The nonaqueous electrolyte is interposed between the positive electrode and the negative electrode. Further, as the case, a known aluminum case or the like usually used as a case of a secondary battery or the like can be used.

According to the secondary battery (energy storage device), since a nonaqueous electrolyte containing halogenated toluene and halogenated nitrotoluene is used, swelling of the case caused by charge-discharge is suppressed. In addition, in the secondary battery, gas accumulation is less likely to occur between the electrode plates, so that the capacity retention ratio is high and the battery life is long.

### (Positive Electrode)

The positive electrode has a positive electrode substrate and a positive active material layer disposed directly or via an intermediate layer on the positive electrode substrate.

The positive electrode substrate has conductivity. As the material of the substrate, metal such as aluminum, titanium, tantalum or stainless steel or an alloy thereof is used. Among them, aluminum and an aluminum alloy are preferable from balance between potential resistance, high conductivity, and cost. Further, examples of formation form of the positive electrode substrate include a foil, a vapor deposited film and the like, and foil is preferable from the viewpoint of cost. That is, aluminum foil is preferable as the positive electrode substrate. Incidentally, examples of aluminum or aluminum alloy include A1085P, A3003P and the like prescribed in JIS-H-4000 (2014).

The intermediate layer is a coating layer on the surface of the positive electrode substrate and contains conductive particles such as carbon particles to reduce contact resistance between the positive electrode substrate and the positive active material layer. The constitution of the intermediate layer is not particularly limited, and the intermediate layer can be formed from a composition containing, for example, a resin binder and conductive particles. Note that having "conductivity" means that the volume resistivity measured in accordance with JIS-H-0505 (1975) is 107 Ω cm or less, and "non-conductive" means that the volume resistivity exceeds 107 Ω cm.

The positive active material layer is formed of a so-called positive electrode mixture containing a positive active material. In addition, the positive electrode mixture forming the positive active material layer contains optional components such as a conductive agent, a binder (binding agent), a thickener or a filler, as necessary.

Examples of the positive active material include composite oxides represented by LixMOy (M represents at least one transition metal) (LixCoO2, LixNiO2, LixMnO3, LixNiαCO(1-α)O2, LixNiαMnβCo(1-α-β)O2 and the like having a layered α-NaFeO2 type crystal structure, LixMn2O4, LixNiαMn(2-α)O4 and the like having a spinel type crystal structure), and polyanion compounds represented by LiwMex(XOy)z (Me represents at least one transition metal, and X represents, for example, P, Si, B, V or the like) (LiFePO4, LiMnPO4, LiNiPO4, LiCoPO4, Li3V2(PO4)3, Li2MnSiO4, Li2CoPO4F, and the like). Elements or polyanions in these compounds may be partially substituted with other elements or anionic species. In the positive active material layer, one of these compounds may be used alone, or two or more of these compounds may be mixed and used.

The conductive agent is not particularly limited as long as it is a conductive material that has no adverse effect on battery performance. Examples of such conductive agent include carbon black such as natural or artificial graphite, furnace black, acetylene black and ketjen black, metal, conductive ceramics, and the like. Examples of the shape of the conductive agent include powder, fiber, and the like.

Examples of the binder (binding agent) include thermoplastic resins such as fluororesins (polytetrafluoroethylene (PTFE), polyvinylidene fluoride (PVDF), etc.), polyethylene, polypropylene and polyimide; elastomers such as ethylene-propylene-diene rubber (EPDM), sulfonated EPDM, styrene butadiene rubber (SBR) and fluorine rubber; polysaccharide polymers; and the like.

Examples of the thickener include polysaccharide polymers such as carboxymethylcellulose (CMC) and methylcellulose. Also, when the thickener has a functional group reactive with lithium, it is preferable to previously deactivate this functional group by methylation or the like.

The filler is not particularly limited as long as it has no adverse effect on battery performance. Examples of the main component of the filler include polyolefins such as polypropylene and polyethylene, silica, alumina, zeolite, glass, carbon and the like.

### (Negative Electrode)

The negative electrode has a negative electrode substrate and a negative active material layer disposed directly or via an intermediate layer on the negative electrode substrate. The intermediate layer can have the same constitution as the intermediate layer of the positive electrode.

The negative electrode substrate may have the same constitution as that of the positive electrode substrate. As the material, metal such as copper, nickel, stainless steel or nickel-plated steel or an alloy thereof is used, and copper or a copper alloy is preferable. That is, copper foil is preferable as the negative electrode substrate. Examples of the copper foil include rolled copper foil, electrolytic copper foil, and the like.

The negative active material layer is formed of a so-called negative electrode mixture containing a negative active material. In addition, the negative electrode mixture forming the negative active material layer contains optional components such as a conductive agent, a binder (binding agent), a thickener or a filler, as necessary. As the optional components such as the conductive agent, the binder, the thickener and the filler, the same materials as those of the positive active material layer can be used.

As the negative active material, a material which can occlude and release lithium ions is usually used. Specific examples of the negative active material include metals or semi-metals such as Si and Sn; metal oxides or semi-metal oxides such as Si oxides and Sn oxides; polyphosphoric acid compounds; graphite, carbon materials such as amorphous carbon (easily graphitizable carbon or non-graphitizable carbon), and the like.

Further, the negative electrode mixture (negative active material layer) contains a typical nonmetallic element such as B, N, P, F, Cl, Br or I, a typical metallic element such as Li, Na, Mg, Al, K, Ca, Zn, Ga or Ge, or a transition metal element such as Sc, Ti, V, Cr, Mn, Fe, Co, Ni, Cu, Mo, Zr, Ta, Hf, Nb or W.

### (Separator)

As the material of the separator, for example, a woven fabric, a nonwoven fabric, a porous resin film or the like is used. Among them, a porous resin film is preferable from the viewpoint of strength, and a nonwoven fabric is preferable from the viewpoint of liquid retaining property of the nonaqueous electrolyte. As the main component of the separator, for example, polyolefin such as polyethylene or polypropylene is preferable from the viewpoint of strength, and polyimide, aramid or the like is preferable from the viewpoint of oxidation degradation resistance. Further, these resins may be combined.

### <Method for Producing Energy Storage Device>

A method for producing an energy storage device according to an embodiment of the present invention is a method for producing a nonaqueous electrolyte secondary battery having a positive electrode, a negative electrode and a nonaqueous electrolyte, and the nonaqueous electrolyte is used as the nonaqueous electrolyte. The method includes a step of housing a positive electrode and a negative electrode (electrode assembly) in a case, and a step of injecting the nonaqueous electrolyte into the case.

The injection can be performed by a known method. After the injection, a secondary battery (energy storage device) can be obtained by sealing an injection port. Details of each element constituting the secondary battery obtained by the method are as described above. According to the method, by using the nonaqueous electrolyte, it is possible to obtain a secondary battery (energy storage device) in which swelling of the case caused by repeated charge-discharge is suppressed.

### <Other Embodiments>

The present invention is not limited to the above-described embodiments, and can be implemented in aspects with various modifications and improvements, besides the above aspects. For example, in the positive electrode or the negative electrode, an intermediate layer may not be provided. In addition, in the above-described embodiments, the embodiment in which the energy storage device is a secondary battery has been mainly described, but other energy storage devices may be used. Examples of other energy storage devices include capacitors (electric double layer capacitors, lithium ion capacitors), and the like.

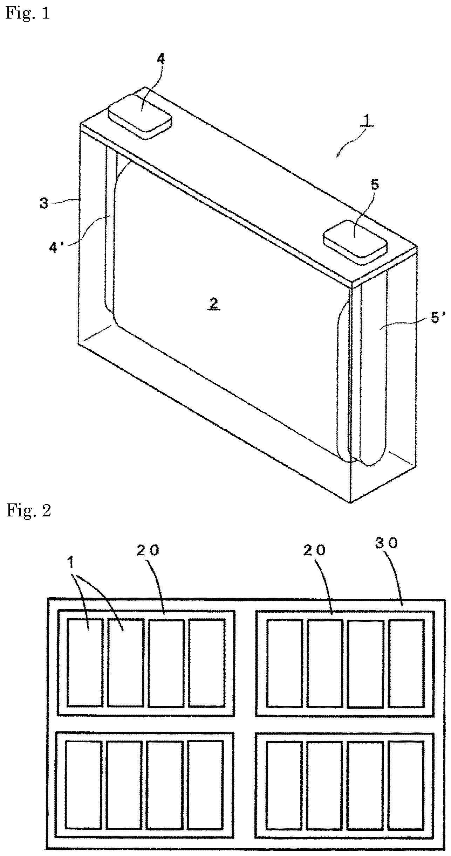

Fig. 1 is a schematic view of a rectangular secondary battery 1 which is an embodiment of an energy storage device according to the present invention. Incidentally, the same figure is a perspective view of the inside of a case. In the lithium secondary battery 1 shown in Fig. 1, an electrode assembly 2 is housed in a battery case 3. The electrode assembly 2 is formed by winding a positive electrode including a positive active material and a negative electrode including a negative active material via a separator interposed therebetween. The positive electrode is electrically connected to a positive electrode terminal 4 via a positive electrode lead 4', and the negative electrode is electrically connected to a negative electrode terminal 5 via a negative electrode lead 5'. In addition, a nonaqueous electrolyte according to one embodiment of the present invention is injected into the battery case 3.

The constitution of the energy storage device according to the present invention is not particularly limited, and examples thereof include a cylindrical battery, a prismatic battery (rectangular battery), a flat battery, and the like. The present invention can also be realized as an energy storage apparatus including a plurality of the above energy storage devices. An embodiment of the energy storage apparatus is shown in Fig. 2. In Fig. 2, an energy storage apparatus 30 includes a plurality of energy storage units 20. Each of the energy storage units 20 includes a plurality of secondary batteries 1. The energy storage apparatus 30 can be mounted as a power source for automobiles such as electric vehicles (EV), hybrid vehicles (HEV) and plug-in hybrid electric vehicles (PHEV).

### EXAMPLES

Hereinafter, the present invention will be described further specifically with reference to examples. However, the present invention is not limited to the following examples.

### [Example 1]

### (Preparation of Nonaqueous Electrolyte)

LiPF6 was dissolved at a concentration of 1.0 M in a solvent in which EC and EMC were mixed at a volume ratio of 30 : 70. To this were further added o-fluorotoluene (OFT) as additive A to be 5% by mass and 2-fluoro-6-nitrotoluene as additive B to be 0.005% by mass, to obtain a nonaqueous electrolyte of Example 1.

### (Preparation of Energy Storage Device)

A positive electrode plate containing LiNi1/3Co1/3Mn1/302 having an α-NaFeO2 type crystal structure as a positive active material was prepared. Further, a negative electrode plate containing graphite as a negative active material was prepared. Subsequently, the positive electrode plate and the negative electrode plate were laminated via a separator made of a polyethylene microporous film, and the electrode assembly was prepared by winding the positive electrode plate and the negative electrode plate in a flat shape. The electrode assembly was housed in an aluminum prismatic container case, and a positive electrode terminal and a negative electrode terminal were attached. After injecting the nonaqueous electrolyte into the case (prismatic container case), the case was sealed to obtain an energy storage device (lithium ion secondary battery).

### [Examples 2 to 3, Comparative Examples 1 to 5]

The same procedure was carried out as in Example 1, except that the types and contents of the additives A and B used were as shown in Table 1, to obtain nonaqueous electrolytes of Examples 2 to 4 and Comparative Examples 1 to 5, and energy storage devices. In the table, "-" indicates that no corresponding additive is added.

### [Evaluation]

### (Charge-Discharge Cycle Test)

For each of the obtained energy storage devices, initial charge-discharge was performed at 25°C with a charge upper limit voltage of 4.2 V and an end-of-discharge voltage of 2.75 V. Subsequently, constant current voltage charge was performed in a constant temperature chamber at 45°C with a charge current of 850 mA, a charge upper limit voltage of 4.20 V, and a total charge time of 3 hours, and then a pause period of 10 minutes was provided. Thereafter, a constant current discharge was performed at a discharge current of 850 mA and an end-of-discharge voltage of 2.75 V, and then a pause period of 10 minutes was provided. This charge-discharge was performed for 500 cycles. After that, charge-discharge was performed at 25°C with a charge upper limit voltage of 4.2 V and an end-of-discharge voltage of 2.75 V.

### (Measurement of Battery Swelling)

The battery thickness was measured in the discharged state when charge-discharge was performed at 25°C after the initial charge-discharge and after 500 cycles in the charge-discharge cycle test. The amount of increase in thickness when charge-discharge was performed at 25°C after 500 cycles with respect to the thickness after the initial charge-discharge was obtained, respectively, and this was defined as battery swelling (mm). The results are shown in Table 1.

### (Capacity Retention Ratio)

The ratio of the discharge capacity when charge-discharge was performed at 25°C after 500 cycles to the discharge capacity at the time of the initial charge-discharge in the charge-discharge cycle test was determined as "capacity retention ratio (%)". The results are shown in Table 1.

**[Table 1]**

| | Additive A | | Additive B | | Evaluation | |
|---|---|---|---|---|---|---|
| | Type | Content | Type | Content | Battery swelling | Capacity retention ratio |
| | - | % by mass | - | % by mass | mm | % |
| Example 1 | OFT | 5 | 2-Fluoro-6-nitrotoluene | 0.005 | 7.1 | 66 |
| Example 2 | OFT | 5 | 2-Fluoro-6-nitrotoluene | 0.05 | 6.9 | 68 |
| Example 3 | OFT | 5 | 2-Fluoro-6-nitrotoluene | 0.1 | 6.8 | 68 |
| Example 4 | OFT | 5 | 2-Fluoro-4-nitrotoluene | 0.1 | 6.9 | 67 |
| Comparative Example 1 | OFT | 5 | - | - | 7.5 | 65 |
| Comparative Example 2 | - | - | 2-Fluoro-6-nitrotoluene | 0.1 | 7.8 | 61 |
| Comparative Example 3 | - | - | 2-Fluoro-6-nitrotoluene | 5 | 9.0 | 50 |
| Comparative Example 4 | - | - | Nitrobenzene | 5 | 8.7 | 56 |
| Comparative Example 5 | - | - | 2-Fluoro-4-nitrotoluene | 0.1 | 7.9 | 60 |

As shown in the above Table 1, it can be seen that battery swelling is reduced in Examples 1 to 4 in which both halogenated toluene and halogenated nitrotoluene were added, as compared with Comparative Example 1 in which only halogenated toluene (OFT) was added as an additive. In addition, it can be seen that the capacity retention ratios of Examples 1 to 4 are also high as compared with those of Comparative Examples 1 to 5. On the other hand, battery swelling of Comparative Examples 2 to 4 in which halogenated toluene was not added and halogenated nitrotoluene or nitrobenzene was added was larger than that of Comparative Example 1, and the capacity retention ratio was also relatively low. Accordingly, it can be seen that the effect of suppressing battery swelling and high capacity retention ratio are effects which are exerted when both halogenated toluene and halogenated nitrotoluene are used. In addition, when comparing Example 3 with Example 4, it can be seen that the battery swelling suppression effect tends to be higher in the case of adding 2-fluoro-6-nitrotoluene than in the case of adding 2-fluoro-4-nitrotoluene. When comparing Comparative Example 2 with Comparative Example 3, it can be seen that battery swelling tends to relatively increase when a large amount of 2-fluoro-6-nitrotoluene is added as the additive B.

### INDUSTRIAL APPLICABILITY

The present invention can be applied to nonaqueous electrolyte energy storage devices such as nonaqueous electric field secondary batteries used as power sources for electronic apparatuses such as personal computers and communication terminals, automobiles and the like, nonaqueous electrolytes provided therefor, and the like.

### DESCRIPTION OF REFERENCE SIGNS

- 1:: Nonaqueous electrolyte secondary battery
- 2:: Electrode assembly
- 3:: Battery case
- 4:: Positive electrode terminal
- 4':: Positive electrode lead
- 5:: Negative electrode terminal
- 5':: Negative electrode lead
- 20:: Energy storage unit
- 30:: Energy storage apparatus

## Claims

1. A nonaqueous electrolyte for an energy storage device in which an electrolyte salt is dissolved in a nonaqueous solvent and which further contains halogenated toluene and halogenated nitrotoluene, wherein the content of the halogenated toluene is 0.1% by mass or more and the content of the halogenated nitrotoluene is 0.001% by mass or more based on a total mass of the nonaqueous electrolyte.

2. The nonaqueous electrolyte according to claim 1, wherein the halogenated toluene is fluorotoluene.

3. The nonaqueous electrolyte according to claim 1 or 2, wherein a content of the halogenated toluene is 0.1% by mass or more and 8% by mass or less based on a total mass of the nonaqueous electrolyte.

4. The nonaqueous electrolyte according to claim 1, 2 or 3, wherein the halogenated nitrotoluene is fluoronitrotoluene.

5. The nonaqueous electrolyte according to any one of claims 1 to 4, wherein a content of the halogenated nitrotoluene is 0.001% by mass or more and 3% by mass or less based on the total mass of the nonaqueous electrolyte.

6. An energy storage device comprising the nonaqueous electrolyte according to any one of claims 1 to 5.

7. A method for producing an energy storage device, the method including a step of housing a positive electrode and a negative electrode in a case, and a step of injecting the nonaqueous electrolyte according to any one of claims 1 to 5 into the case.

## Patentansprüche

1. Nichtwässriger Elektrolyt für eine Energiespeichervorrichtung, bei dem ein Elektrolytsalz in einem nichtwässrigen Lösungsmittel gelöst ist und der weiterhin halogeniertes Toluol und halogeniertes Nitrotoluol enthält, wobei der Gehalt an dem halogenierten Toluol 0,1 Masse-% oder mehr beträgt und der Gehalt an dem halogenierten Nitrotoluol 0,001 Masse-% oder mehr beträgt, bezogen auf eine Gesamtmasse des nichtwässrigen Elektrolyten.

2. Nichtwässriger Elektrolyt nach Anspruch 1, wobei das halogenierte Toluol Fluortoluol ist.

3. Nichtwässriger Elektrolyt nach Anspruch 1 oder 2, wobei ein Gehalt an dem halogenierten Toluol 0,1 Masse-% oder mehr und 8 Masse-% oder weniger, bezogen auf eine Gesamtmasse des nichtwässrigen Elektrolyten, beträgt.

4. Nichtwässriger Elektrolyt nach Anspruch 1, 2 oder 3, wobei das halogenierte Nitrotoluol Fluornitrotoluol ist.

5. Nichtwässriger Elektrolyt nach einem der Ansprüche 1 bis 4, wobei ein Gehalt an dem halogenierten Nitrotoluol 0,001 Masse-% oder mehr und 3 Masse-% oder weniger, bezogen auf die Gesamtmasse des nichtwässrigen Elektrolyten, beträgt.

6. Energiespeichervorrichtung, umfassend den nichtwässrigen Elektrolyten nach einem der Ansprüche 1 bis 5.

7. Verfahren zur Herstellung einer Energiespeichervorrichtung, wobei das Verfahren einen Schritt des Unterbringens einer positiven Elektrode und einer negativen Elektrode in einem Gehäuse und einen Schritt des Injizierens des nichtwässrigen Elektrolyten nach einem der Ansprüche 1 bis 5 in das Gehäuse umfasst.

## Revendications

1. Electrolyte non aqueux destiné à un dispositif de stockage d'énergie dans lequel un sel électrolytique est dissout dans un solvant non aqueux et qui contient en outre du toluène halogéné et du nitrotoluène halogéné, dans lequel la teneur en toluène halogéné est égale ou supérieure à 0,1 % en masse et la teneur en nitrotoluène halogéné est égale ou supérieure à 0,001 % en masse, sur la base d'une masse totale d'électrolyte non aqueux.

2. Electrolyte non aqueux selon la revendication 1, dans lequel le toluène halogéné est du fluoro toluène.

3. Electrolyte non aqueux selon la revendication 1 ou la revendication 2, dans lequel une teneur en toluène halogéné est égale ou supérieure à 0,1 % en masse et inférieure ou égale à 8 % en masse, sur la base d'une masse totale d'électrolyte non aqueux.

4. Electrolyte non aqueux selon la revendication 1, la revendication 2 ou la revendication 3, dans lequel le nitrotoluène halogéné est du fluoro nitrotoluène.

5. Electrolyte non aqueux selon l'une quelconque des revendications 1 à 4, dans lequel une teneur en nitrotoluène halogéné est égale ou supérieure à 0,001 % en masse et inférieure ou égale à 3 % en masse, sur la base de la masse totale d'électrolyte non aqueux.

6. Dispositif de stockage d'énergie comprenant l'électrolyte non aqueux selon l'une quelconque des revendications 1 à 5.

7. Procédé de production d'un dispositif de stockage d'énergie, le procédé comportant une étape de logement d'une électrode positive et d'une électrode négative dans un compartiment, et une étape d'injection de l'électrolyte non aqueux selon l'une quelconque des revendications 1 à 5 dans le compartiment.
